# EUROPEAN PATENT SPECIFICATION

(11) **EP 0 975 379 B1**
(45) Date of publication and mention of the grant of the patent: **30.10.2002**
(21) Application number: 98912525.7
(22) Date of filing: 30.03.1998
(51) Int. Cl.: A61L 27/00, A61K 6/097

(54) **A BIOCOMPATIBLE COMPOSITION, METHODS FOR ITS PREPARATION AND USES THEREOF**
BIOVERTRÄGLICHES MITTEL, METHODE SEINER HERSTELLUNG UND SEINES GEBRAUCHS
COMPOSITION BIOCOMPATIBLE, PROCEDES PERMETTANT DE LA PREPARER ET SES UTILISATIONS

(30) Priority: 04.04.1997 FI 971385
(43) Date of publication of application: 02.02.2000
(73) Proprietor: Bioxid Oy, 20520 Turku (FI); Cellomeda Oy, 20520 Turku (FI)
(72) Inventor: ANDERSSON, Örjan, FIN-65280 Vasa (FI); LÖNNQVIST, Kurt, FIN-20500 Abo (FI); YLI-URPO, Antti, FIN-20660 Littoinen (FI); VILJANTO, Jouko, FIN-20720 Turku (FI)
(74) Representative: Öhman, Ann-Marie
(86) International application number: FI9800283
(87) International publication number: WO98044964

(56) References cited:
- EP-A- 0 714 666
- WO-A-86/01113

## Description

### FIELD OF THE INVENTION

This invention relates to a novel biocompatible composition comprising a bioactive material added to a porous spongy cellulose material, which is bioresorbable. Furthermore, the invention relates to methods for the preparation of said composition and to uses thereof.

### INTRODUCTION AND BACKGROUND OF THE INVENTION

The publications and other materials used herein to illuminate the background of the invention, and in particular, cases to provide additional details respecting the practice, are incorporated by reference.

A bioactive material is biocompatible and has affinity for living tissue. Traditional bioactive materials, such as bioactive glass and hydroxylapatite are rigid. These can be used as bulk specimens, coatings or as granules or powders. An advantage of granules and powders is that they can be used in irregular bone defects or tissue structures. Thus, bioactive glass and hydroxylapatite granules are used to replace bone and to fill bony defects. Granules can also be used around teeth to stabilise them.

Considering bioactive materials such as bioactive glass granules as an example, these have some advantages and some disadvantages. The advantages are easy handling of the granules and good osteoconductivity and bone bonding ability. A major disadvantage which limits the use of bioactive materials is their lack of flexibility. A solid glass implant, for example, cannot be bent to fit an irregular defect. If granules are used there is a risk that they may migrate from the implantation site. Even if a solid piece of glass can be shaped with a diamond burr, it would be advantageous to have a material which simply could be cut with a pair of scissors in the operating theatre. In order to achieve a good fit in the implantation site it would be an advantage if the material would expand after being put into place. This cannot be achieved with known bioactive glasses, hydroxylapatite, or any other known bioactive material. Another disadvantage of bioactive glass in some applications is their slow resorption. An ideal implant material would only provide a scaffold for bone growth and then disappear when the healing is complete. A bioactive glass which has proven successful as a bone filler in the form of granules is the one called S53P4 (1). However, the glass is only slowly resorbed when used as granules of 300 - 500 µm size. If the particle size is reduced, the surface area is correspondingly increased and a more rapid dissolution or resorption follows. However, smaller particles give stronger increase in pH, which may change the surface reactions of the glass in a direction unfavourable for bone formation. Therefore, it appears difficult to increase the resorption rate of the bioactive glass without compromising the biological performance.

### OBJECTS OF THE INVENTION

The object of the present invention is a composition providing a solution to the above discussed draw-backs of bioactive glass and other known bioactive materials as well as implants made thereof.

In comparison with traditional bioactive implant materials the present invention provides a number of advantages:
- The composition is flexible and can be compressed and fitted to irregular bone defects.
- The composition can be cut to appropriate size and shape in the operation theatre, using a pair of scissors or a scalpel.
- The composition can be made in versions that swell after implantation and thus, becomes immediately fixed.
- The spongy structure of the composition and the implant made thereof facilitates bone ingrowth and eventually total replacement of the implant with bone.
- The composition provides a sufficiently small total surface area and pH increase even if small (thus rapidly resorbing) glass particles are used.

Additional advantages of the composition according to the invention are:
- The composition is easy to handle.
- The composition can be made in a form that acts as a source for soluble silicon. Soluble silicon has been reported to stimulate bone formation. In addition to being essential for the bonding of a bioactive glass to bone, silica is also a constituent of many biological systems (2). Carlisle showed that silica is essential for the development of bone (3). Recent reports suggest metabolic function (4) and intracellular mitogenic effects (5) of soluble silicon. These aspects were recently discussed by Hench (6).
- The release rate can be controlled by choice of process parameters or by choice of appropriate bioactive material (e.g. choice of compositions of glass or gel).
- As a silicic acid releasing device, the composition can be used for treatments involving remineralisation or mineralisation of tissue, for example of dentine.

### SUMMARY OF THE INVENTION

Thus, according to one aspect this invention provides a biocompatible composition, wherein said composition comprises a porous spongy material of a bioresorbable cellulose with hydroxyl- and/or carboxylic acid groups, and a bioactive material,
- wherein said bioactive material is distributed within said porous material according to claim 1.

According to another aspect this invention provides an implant or a device for use in the treatment of sensitive teeth comprising a composition according to this invention. Said implant or device has the form of
- fibres,
- non-woven granules or pellets, a non-woven block, rod, sheet, membrane, tread, filter, foil, tube or coating; or
- a woven structure such as a sheet, a membrane, a filter, a sock, a tube or a coating.

Furthermore, this invention concerns a method for the preparation of a composition as defined above, said method comprising the steps of
- mixing particles of the bioactive material with a slurry of fibres of a cellulose material and one or more auxiliary substances necessary to convert said fibres into a porous spongy material, and
- allowing said fibres and said auxiliary substance or substances to form the porous spongy material.

According to yet another aspect, this invention describes a method for the preparation of a composition as defined above, said method being characterized in that the porous material of a bioresorbable organic polymer with hydroxyl- and/or carboxylic acid groups is immersed into a solution containing a silicon compound, whereafter the silicon is brought into the form of a gel, after which the immersed product optionally is acidified, pressed (or pressed, acidified) and dried.

According to yet another aspect, this invention concerns a method for the preparation of a composition as defined above, said method being characterized in that
- particles of the bioactive material are mixed with a slurry of fibres of a cellulose material and one or more auxiliary substances necessary to convert said fibres into a porous spongy material,
- said fibres and said auxiliary substance or substances are allowed to form the porous spongy material,
- the porous material containing particles of bioactive material is immersed into a solution containing a silicon compound, whereafter the silicon is brought into the form of a gel, after which the immersed product optinonally is acidified and then pressed and dried.

Furthermore, this invention describes the use of a composition or an implant or a device as defined above for replacement of bone and/or filling of bone defects, for mineralisation of tissue such as dentine for tretment of sensitive teeth, for stabilisation of teeth, for stabilisation of an implant, for replacement or augmentation of cartilage or for use in soft tissue surgery.

Furthermore, this invention describes the use of a composition or an implant or a device as defined above for clinical applications to replace bone grafts to 1) substitute bone defects in all areas of orthopedics, neurosurgery, ENT-surgery and dentistry; 2) stimulate fracture healing, especially in case of pseudoarthrosis; 3) to create cancellous bone in soft tissues for grafting purposes; or 4) to speed up osteosynthesis.

Furthermore, this invention concerns the use of a composition or an implant as defined above or an implant as a carrier for drug delivery.

### DETAILED DESCRIPTION OF THE INVENTION

In the following the term "biocompatible composition" shall be understood to mean a composition that is fysiologically acceptable when brought into contact with body fluids and body tissue.

"Bioactive materials" refer to materials that are able to bind with body tissue and shall be understood to cover bioactive glasses, bioactive ceramics, glass ceramics and silica gels.

Bioactive glasses refer to amorphous mixtures of Si-oxide or Si-hydroxide with oxides of one or more elements such as sodium, potassium, calcium, magnesium, boron, titanium, aluminium and phosphorus. Some of the typical bioactive glass compositions are presented in Table 1.

**Table 1.**

| Composition (weight-%) of some bioactive glasses | | | | | | |
|---|---|---|---|---|---|---|
| Designation | Na₂O | CaO | P₂O₅ | B₂O₃ | Al₂O₃ | SiO₂ |
| S53P4 | 23.00 | 20.00 | 4.00 | 0.00 | 0.00 | 53.00 |
| S45P7 | 24.00 | 22.00 | 7.00 | 2.00 | 0.00 | 45.00 |
| S55.5P4 | 29.00 | 11.00 | 4.00 | 0.00 | 0.50 | 55.50 |

Bioactive ceramics are crystalline materials such as calcium phosphates and calcium carbonate.

Bioactive glass ceramics are composites comprising crystals embedded in an amorphous phase.

The wording "porous material" referred to hereinafter is a material enabling blood vessels to penetrate the same. Therefore, the material shall have an open pore structure and a pore diameter of at least 100 µm, preferably at least 500 µm (7).

The organic polymer forming the bioresorbable porous material is a polysaccaride such as cellulose, a cellulose derivative, partially decomposed cellulose or cellulose derivative, starch, and the like.

The compositions containing the bioactive material mainly in the form of particles is called a composition of "Type A" in the following. The bioactive material in the form of particles is preferably a bioactive glass, e.g. one of those listed in Table 1. The bioactive glass coded S53P4 is particularly preferred.

The compositions comprising bioactive material in the form of a Si-gel derived from a solution containing a silicon compound is in the following called a "Type B" composition. This composition could optionally also contain bioactive material in the form of particles. Thus, a Type A composition can be treated with a solution containing a silicon compound to give a Type B composition.

It should be noted that both compositions (Type A and Type B), although differing in structure and manufacturing, have similar properties and can be used for similar purposes.

The ratio of bioactive material : amount of porous material should preferably be at least 1:1, most perferably about 4:1.

In a Type A composition the amount of bioactive particles should preferably vary in the range 50 - 99.9 weight-%.

### Description of a Type A composition

In developing a Type A composition containing bioactive glass as the bioactive material and cellulose as the porous material a number of different particle size fractions and amounts of glass were studied. The particles could not be introduced into the finished porous material of cellulose, but they had to be added during the stage of manufacturing of the porous cellulose material from its raw materials. The manufacturing process had to be optimised in order to avoid adverse chemical changes in the glass, which could have potential negative effects on the biological performance. The optimum particle amount was chosen on basis of manufacturing aspects and aspects of particle-to-particle distances. The resulting composition can be easily cut to appropriate size and shape, it is flexible and it expands by absorbing water or tissue fluid. The content of inorganic bioactive material is sufficient to have a stimulatory effect on bone growth, yet low enough to facilitate sufficiently rapid resorption.

The retention of particles in the cellulose is generally good, although some particles may loosen when the material is cut. By acid tratment the surface of the glass particles can be transformed to silica gel with a high surface concentration of hydroxyl groups. It should be recognised that one special feature of cellulose is its strong tendency to form hydrogen bonds with neighbouring cellulose molecules. Also intramolecular hydrogen bonds form. Thus, in addition to mechanical retention, the hydrogen bonding capacity of the cellulose also facilitates attachment between cellulose and the Si-gel surface of bioactive glass particles. Another method to further improve the retention of the glass particles in the cellulose is by causing a Si-gel to be formed therein, said Si-gel having been derived from a solution of a silicon compound. This is discussed later as a combination of the compositions of Type A and Type B according to the present invention.

### Description of a Type B composition

Compositions of Type B comprise a porous material e.g. based on cellulose which after their normal manufacturing have been given an additional treatment with a soulution containing a silicon compound. Thus, the cellulose material is immersed in a solution which contains silicon in the form of silicate ions (water-glass), silicic acid or silica gel (e.g. acidified water-glass) or in the form of organic silicon containing compounds (sol-gel precursors). Common to all silicon sources is that they are subsequently treated to transform them into silica gel.

The high pH of alkali silicate solutions (water-glass) may have negative effects on the biological response. By acidification Si-gel forms and the pH of the material is reduced. Silicic acid can also be made to precipitate by adjusting pH or by adding ions such as calcium. The organic silicon compounds may be transformed to silica gel by the sol-gel process. Regardless of the source of silicon and the manufacturing technique, the final composition consists of a cellulose material impregnated with silica gel. The Si-gel serves two purposes. Si-gel is known to be highly biocompatible and thus, to have advantages as an implant material. Si-gel also dissolves slowly in the tissue and silicic acid is released. In addition to being essential for the bonding of a bioactive glass to bone, silica is also a constituent of many biological systems (2). Carlisle showed that silica is essential for the development of bone (3). Recent reports suggest metabolic function (4) and intracellular mitogenic effects (5) of soluble silicon. These aspects were recently discussed by Hench (6).

The capacity of the Type B composition to release soluble silicon can also be used in applications were silicic acid or silicate ions are to be transferred into tissue in order to induce mineralisation. One such example is the use of composition B as a device for treating sensitive teeth (with open dentine tubules), described in (8).

It shall be stressed that the experimental findings for the Type A and Type B compositions reported here also will apply to other compositions of these types where cellulose.

### Type C composition

According to this alternative, the porous material of the organic polymer is shaped to a container, which can be flexible or stiff. Said container is then filled with particles of or a gel of the bioactive material.

### Manufacturing of Type A compositions

In combining a highly porous, flexible and expandable cellulose material with a bioactive particulate material several problems had to be solved. In the below referred work the bioactive particulate material used was bioactive glass and the porous material was a cellulose sponge. The glass must not undergo adverse changes in the manufacturing process. Thus, both the glass and the composite manufacturing process must be chosen so that problems are avoided. The glass must also show sufficient retention within the cellulose material. Otherwise, the composition will not remain isotropic, but bioactive particles may be lost in some parts of the composite due to mechanical loads. To a small extent particles may loosen from Type A composition during cutting in the dry state. However, no changes in the particle distribution within the composite were observed. Thus, the composition was isotrope.

In compositions of Type A the bioactive particles are introduced in a slurry of bioresorbable organic cellulose fibres as defined before, and one or more auxiliary substances useful to convert said fibres into a porous material. In this way the organic molecules can attach around the irregular shape of the particles. Through hydrogen bonding the retention is probably improved as compared to particulate materials and organic materials without the exceptionally large amounts of hydroxyl groups characteristic of cellulose and Si-gel.

### Manufacturing of a Type B composition

The first version of Type B composition was developed by using a sodium silicate solution (water-glass) and a viscose cellulose sponge. A cellulose sponge is particularly advantageous due to its high porosity. The following parameters were studied:
- different dilutions of water-glass, i.e. different silicate ion concentrations and pH
- the amount of water-glass in the sponge (pressed or not pressed after immersion in water glass)
- the role of acidification (strength and volume of acid used)
- the drying process (in air at room temperature, at elevated temperature, freeze-drying)

Several problems were encountered. By immersing the sponge in the alkaline silicate solution (water-glass) and subsequent drying, a composition was obtained that contained a great amount of silicate ions. When immersed in an aqueous solution or in living tissue the dissolving sodium silicate strongly increased the pH in its surroundings. This is not acceptable from a biocompatibility point of view. The problem with the alkaline effect of the implant should in principle be possible to avoided by acidifying the water glass containing cellulose material. However, when the alkaline water-glass containing sponge was immersed in the acid, the water glass dissolved into the acid and formed Si-gel in the solution, rather than in the sponge. By experiments with different acid strengths and volumes it was possible to avoid the extensive dissolution of silicate ions into the acid solution. Instead the Si-gel precipitated within the sponge. The Si-gel impregnated sponge was subsequently dried. Also the drying process had to be optimised, since the Si-gel dried very slowly at room-temperature and humidity. This is not surprising since Si-gel can be used as a desiccant due to its water absorbing capacity. The method finally chosen was drying at a slightly elevated temperature.

The compositions according to the invention and their preparation is described more in detail in the following non-limiting examples.

### Example 1

### Preparation of a Type A composition

A cellulose viscose solution containing 5 % of cellulose was prepared according to known methods. Calculated on the weight the cellulose, 20 % of cotton fibres cut to 8 - 10 mm lenght was mixed into the viscose. A pore-forming material Na₂SO₄ . 10 H₂O (Glauber's salt) with a particle size < 1.0 mm was used. This Glauber's salt was mixed with bioactive glass having a grain size of 0,14 - 0,20 mm. The bioactive glass used was the glass designated S53P4 in Table 1. Pore-forming material was added 80 times and bioactive glass 5 times the initial weight of the cellulose. The material obtained was placed into a mold and the cellulose was regenerated by heating in a waterbath. The crude cellulose sponge containing bioactive glass leaving the waterbath was washed salt free by means of hot water, treated with dilute acid, bleaching solution and finally washed repetitively with destilled water. The product was then dried, cut to desired size and packed.

### Example 2

### Preparation of a Type B composition

A cellulose sponge, without bioactive glass, was prepared according to example 1. The cellulose sponge was soaked in a 15 % solution of water glass, transferred to an acid solution, removed therefrom, dried, cut to desired size and packed.

### Example 3

### Preparation of a Type B composition comprising particles of a bioactive material

A cellulose sponge was prepared according to example 1. The cellulose sponge containing bioactive glass was soaked in a 6 % solution of water glass, transferred to an acid solution, removed therefrom, dried, cut to desired size and packed.

### In vitro tests

The materials were tested by immersion in simulated body fluid (SBF). This solution contains inorganic ions in concentration close to those on plasma. The solution was Tris-buffered with 50 mM tris(hydroxylmethyl-aminomethane) and 45 mM HCl. The solution pH was 7.4 and the temperature 37 °C. Test periods were 1, 3, 7, and 14 days. After the test, the samples were removed, dried and prepared for scanning electron microscopy. It was found that the sponges which had been treated with diluted water-glass solutions (total silica content of 3, 6 or 15 %) lost most of their Si-gel within some days. Thus, rapid short-term release was achieved. For the high concentration water-glass (30 %, i.e not diluted) treated sponges surprising phenomena were observed. For the sponges which had not been pressed in order to remove excess water-glass prior to acidification, Si-gel was evenly distributed within the structure of the cellulose sponge. The Si-amount in the sponge decrease with time during the 14 day dissolution test. For the sponges which had been pressed to remove excess water-glass prior to acidification, a dense looking silicon containing layer formed. This layer did not change much during the 14 day dissolution test, as observed by scanning electron microscopy. Thus, pressing the high concentration water-glass treated sponge resulted in a composition with a lower total Si-content and a slower release of this content as compared with the corresponding not pressed sponge. The latter sponge had a higher amount of Si-gel prior to dissolution testing, but a lower amount after the 14 day dissolution test. Thus, for the same water-glass concentration in the immersion solution, the Si-release rate of the resulting composition can be adjusted by changing the extent to which the cellulose material is pressed. A high rate, high total amount, short-term release device as well as a low rate, lower total amount, long-term release device can be obtained. Devices with intermediate release rates, total amounts and life times (i.e. release duration) can be obtained by varying the extent to which the cellulose material is pressed prior to acidification.

### Advantages of the composition

When the cellulose material has been impregnated with water-glass, acidified and dried it has become somewhat rigid. The mechanical strength reduces the risk for movements/flexing and associated cracking and loosening of Si-gel fragments. The implant softens if immersed in water or saline prior to implantation. In the wet state it can be easily compressed and fitted into irregular implantation sites.

A particularly advantageous and surprising feature of the composition according to this invention is that it can be compressed and shaped also in its dry state. Thus, after drying, the composition can be shaped for example to a thin plate. The compressed shape remains until the implant is moistened before or after implantation. Thin plates, foils, membranes, filters can be prepared in the wet state and further shaped to e.g tubes in the dry state.

A likewise advantageous and surprising feature is that even the cellulose with the highest Si-gel content, could easily be cut with a pair of scissors or a scalpel. In fact, cutting the Si-gel containing compositions was easier than cutting the pure cellulose sponge.

The wet process can be realised both as a batch process and a continuos one. The continuos process may be similar to that in a paper machine. The cellulose material is collected from the solution on a wire mesh, transported by rollers through an alkali silicate bath, followed by an acid bath (saturated with soluble silicon) and finally it is dried. The composition can also be cut to appropriate shape and size in its moist state.

Compositions of the present invention can be prepared in many forms such as non-vowen blocks, rods, granules/pellets, sheets, threads, membranes, filters, tubes and coatings. The composition can also be in the form of fibres and vowen structures such as sheets, membranes, filters, socks, tubes and coatings.

### Modification of compositions of Type A and Type B

An additional advantage of the compositions according to the invention, is that the cellulose can be broken down (e.g. by oxidative bleaching or by radiation) to different extents, either before or after the addition of the bioactive material. This will result in different degradation rates in the tissue. Thus, the degradation rate for the cellulose can be adjusted to fit that of the Si-release duration of the composition of Type B or the bioactive particle resorption rate of composition of Type A. Similarly, the resorption of the inorganic bioactive material (e.g. glass or gel) can be adjusted by choice of composition, manufacturing parameters, particle size and amount of said material.

The compositions of Type A and Type B can be used as such or combined with biologically or therapeutically active molecules and substances. Such active substances may for example be drugs, antibiotics, hormones, growth factors or other organic compounds. Also inorganic ions may be incorporated into the composition. The inorganic ions can either attach to the cellulose due to its ion exchange capacity, or they may be incorporated into the Si-gel. Inorganic ions may have beneficial effects on bone mineralisation as well as on mineralisation of the implant material. By doping the gel in its manufacturing stage it is also possible to affect its resorption rate. As examples of doping agents can be mentioned stabilising ions such as calcium, magnesium and iron.

The mechanical properties can be affected by introducing some reinforcing or modifying phase. In the present work cotton fibres were used. Other example of reinforcing materials are other cellulose fibres, synthetic biodegradable fibres based on polylactide, polyglycolide, polyhydroxybutyrate, or synthetic inorganic fibres, filaments or whiskers such as glass or Si-gel fibres prepared by the sol-gel method. Common to all fibres is that they with time should resorb.

The manufacturing process used for composition B according to the present invention can be applied as an additional treatment for composition Type A according to the present invention, Thus, the glass particle containing cellulose (Type A) is treated with water glass or another Si-containing solution during or after manufacturing. The precipitated Si-gel basically provides the same advantages as in compositions of Type B, i.e. controlled Si-release and biocompatible surfaces for tissue growth. Additionally the Si-gel further improves the adhesion between glass particles and the cellulose fibres. By animal experiments it was found that the water-glass had no adverse effects on the tissue reactions when used as a glue between particles of bioactive glass.

In the experimental work described above a cotton reinforced viscose cellulose sponge was used. However, the present invention is not restricted to using these materials. A person familiar with the field will recognise that the present invention also can be applied to other cellulose materials or modifications. The Si-impregnation technique can also be used to impregnate other biocompatible and bioresorbable porous material with some affinity for silica gel. The many hydroxyl groups of the cellulose molecule facilitates the attachment of Si-gel by hydrogen bonding. Other biocompatible and bioresorbable organic materials with hydroxyl or carboxylic acid groups are expected to perform in the same way.

### Uses for the compositions according to the invention

Compositions of Type A, Type B, Type C and their combination can be used in many different applications. The multi-purpose feature and the many additional advantages of the invention makes it different from known materials. Some examples of applications are given below.
- Replacement of bone and filling of bony defects
- Use for mineralisation of tissue, e.g. of dentine as a treatment for sensitive teeth (8).
- Use for stabilisation of teeth (composition placed around single teeth)
- Use for stabilisation of implants (placed between the implant and the tissue)
- Use for replacement or augmentation of cartilage (joint surgery, maxillofacial and plastic surgery)
- Use in soft tissue applications
- Use as drug delivery device or as a part thereof
- Use in clinical applications instead of bone grafts to 1) substitute bone defects in all areas of orthopedics, neurosurgery, ENT-surgery and dentistry; 2) stimulate fracture healing, especially in case of pseudoarthrosis; 3) to create cancellous bone in soft tissues for grafting purposes; or 4) to speed up osteosynthesis.

## Claims

1. A biocompatible composition comprising a bioactive material and a porous material, **characterized in that** said bioactive material is distributed within said porous material wherein
i) said bioactive material is present as particles embedded in said porous material and/or
ii) said bioactive material is present as a Si-gel derived from a solution containing a silicon compound, said solution having been brought into contact with the porous material,
and **in that** said porous material is a bioresorbable spongy cellulose material with hydroxyl- and/or carboxylic acid groups, shaped to a reservoir encompassing particles of or a gel of said bioactive material.

2. The composition according to claim 1 **characterized in that** the bioactive material comprises particles of a bioactive glass.

3. The composition according to claim 1 or 2 **characterized in that** the solution containing the silicon compound is an alkali silicate solution.

4. The composition according to claim 1 **characterized in that** the porous material comprising particles of the bioactive material has been immersed into a solution containing a silicon compound, whereafter the silicon has been brought into the form of a gel.

5. The composition according to claim 4 **characterized in that** the porous material is a cellulose sponge, the bioactive material is bioactive glass and the solution containing the silicon compound is an alkali silicate solution.

6. The composition according to any of claims 1 to 5 **characterized in that** the Si-gel is doped with one or more agents able to affect the properties, such as the resorption rate of the Si-gel.

7. The composition according to any of the claims 1 to 6 **characterized in that** said composition is reinforced with organic and/or inorganic fibers.

8. The composition according to any of the claims 1 to 7 **characterized in that** it comprises one or more therapeutically or biologically active agents.

9. An implant comprising a composition according to any of the foregoing claims **characterized in that** said implant has the form of
- fibers,
- non-woven granules or pellets, a non-woven block, rod, sheet, membrane, thread filter, foil, tube or coating; or
- - a woven structure such as a sheet, a membrane, a filter, a sock, a tube or a coating.

10. A method for the preparation of a composition according to claim 1, **characterized in that**
- particles of the bioactive material are mixed with a slurry of fibers of the cellulose material and one or more auxiliary substances necessary to convert said fibers into a porous spongy material, and
- said fibers and said auxiliary substance or substances are allows to form the porous spongy material.

11. The method according to claim 10, **characterized in that** the porous spongy material is further immersed into a solution containing a silicon compound, whereafter the silicon is brought into the form of a gel, after which the immersed product is optionally acidified, pressed and dried.

12. Use of a composition according to any of the claims 1 to 8 for the manufacture of a preparation for replacement of bone and/or filling of bone defects, for mineralisation of tissue such as dentine for treatment of sensitive teeth, for stabilisation of teeth, for stabilisation of implants, for replacement or augmentation of cartilage or for use in soft tissue surgery.

13. Use of a composition according to any of the claims 1 to 8 as a carrier for drug delivery.

14. Use of a composition according to any of the claims 1 to 8 for the manufacture of a preparation for clinical applications to replace bone grafts to 1) substitute bone defects in all areas of orthopedics, neurosurgery, ENT-surgery and dentistry; 2) stimulate fracture healing, especially in case of pseudoarthrosis; 3) to create cancellous bone in soft tissues for grafting purposes; or 4) to speed up osteosynthesis.

15. A device for use in the treatment of sensitive teeth, said device comprising a composition according to any of the foregoing claims **characterized in that** said device has the form of
- fibers,
- non-woven granules or pellets, a non-woven block, rod, sheet, membrane, thread filter, foil, tube or coating; or
- a woven structure such as a sheet, a membrane, a filter, a sock, a tube or a coating.

## Patentansprüche

1. Bioverträgliche Zusammensetzung, umfassend ein bioaktives Material und ein poröses Material, **dadurch gekennzeichnet, dass** das bioaktive Material innerhalb des porösen Materials verteilt ist, wobei
i) das bioaktive Material als Teilchen, eingebettet in dem porösen Material, vorhanden ist und/oder
ii) das bioaktive Material als Si-Gel vorhanden ist, das sich von einer Lösung ableitet, die die Siliciumverbindung enthält, wobei die Lösung in Kontakt mit dem porösen Material gebracht worden ist,
und dass das poröse Material ein bioresorbierbares schwammartiges Cellulosematerial mit Hydroxyl- und/oder Carboxylgruppen ist, geformt zu einem Reservoir, das Teilchen aus bioaktivem Material oder ein Gel aus bioaktivem Material umgibt.

2. Zusammensetzung nach Anspruch 1, **dadurch gekennzeichnet, dass** das bioaktive Material Teilchen aus bioaktivem Glas umfasst.

3. Zusammensetzung nach Anspruch 1 oder 2, **dadurch gekennzeichnet, dass** die Lösung, die die Siliciumverbindung enthält, eine Alkalisilicatlösung ist.

4. Zusammensetzung nach Anspruch 1, **dadurch gekennzeichnet, dass** das poröse Material, das Teilchen aus bioaktivem Material umfasst, in eine Lösung, die eine Siliciumverbindung enthält, eingetaucht wurde, und wobei danach das Silicium in Gelform gebracht wurde.

5. Zusammensetzung nach Anspruch 4, **dadurch gekennzeichnet, dass** das poröse Material ein Celluloseschwamm ist, das bioaktive Material bioaktives Glas ist und dass die Lösung, die die Siliciumverbindung enthält, eine Alkalisilicatlösung ist.

6. Zusammensetzung nach einem der Ansprüche 1 bis 5, **dadurch gekennzeichnet, dass** das Si-Gel mit einem oder mehreren Mitteln, die die Eigenschaften, wie Resorptionsrate des Si-Gels, beeinflussen, dotiert ist.

7. Zusammensetzung nach einem der Ansprüche 1 bis 6, **dadurch gekennzeichnet, dass** die Zusammensetzung mit organischen und/oder anorganischen Fasern verstärkt ist.

8. Zusammensetzung nach einem der Ansprüche 1 bis 7, **dadurch gekennzeichnet, dass** sie ein oder mehrere therapeutisch oder biologisch aktive Mittel umfasst.

9. Implantat, umfassend eine Zusammensetzung nach einem der vorhergehenden Ansprüche, **dadurch gekennzeichnet, dass** das Implantat die Form von
- Fasern,
- nicht-gewebten Granulaten oder Pellets, nichtgewebtem bzw. nicht-gewebter Block, Stab, Film, Membran, Fadenfilter, Folie, Röhre oder Beschichtung; oder
- gewebter Struktur, wie ein Film, eine Membran, ein Filter, eine Hülse, ein Rohr oder eine Beschichtung besitzt.

10. Verfahren zur Herstellung einer Zusammensetzung nach Anspruch 1, **dadurch gekennzeichnet, dass**
- Teilchen aus bioaktivem Material mit einer Aufschlämmung aus Fasern des Cellulosematerials und einer oder mehreren Hilfssubstanzen, die erforderlich sind, um die Fasern in poröses schwammartiges Material zu überführen, vermischt werden und
- die Fasern und die Hilfssubstanz oder Substanzen ein poröses schwammartiges Material bilden gelassen werden.

11. Verfahren nach Anspruch 10, **dadurch gekennzeichnet, dass** das poröse schwammartige Material weiter in eine Lösung eingetaucht wird, die eine Siliciumverbindung enthält, wonach das Silicium in Gelform gebracht wird, nachdem das eingetauchte Produkt gegebenenfalls angesäuert, gepresst und getrocknet wurde.

12. Verwendung einer Zusammensetzung nach einem der Ansprüche 1 bis 8 zur Herstellung eines Präparats für den Ersatz von Knochen und/oder Füllungen von Knochenfehlern für die Mineralisierung von Gewebe, wie Dentin, für die Behandlung empfindlicher Zähne, für die Stabilisierung von Zähnen, für die Stabilisierung von Implantaten, für den Ersatz oder die Vermehrung von Knorpel oder für die Verwendung in der Bindegewebechirurgie.

13. Verwendung einer Zusammensetzung nach einem der Ansprüche 1 bis 8 als Träger für die Arzneimittelabgabe.

14. Verwendung einer Zusammensetzung nach einem der Ansprüche 1 bis 8 für die Herstellung eines Präparats für klinische Anwendungen zum Ersatz von Knochentransplantaten, 1) um Knochenfehler auf allen Gebieten der Orthopädie, der Neurochirurgie, der ENT-Chirurgie und der Zahnmedizin zu substituieren; 2) zur Stimulierung der Frakturheilung, insbesondere im Falle der Pseudoarthrose; 3) zur Erzeugung von spongiösen Knochen in Bindegewebe für Transplantationszwecke; oder 4) zur Beschleunigung der Osteosynthese.

15. Vorrichtung für die Verwendung bei der Behandlung empfindlicher Zähne, wobei die Vorrichtung eine Zusammensetzung nach einem der vorhergehenden Ansprüche umfasst, **dadurch gekennzeichnet, dass** die Vorrichtung die Form von
- Fasern,
- nicht-gewebten Granulaten oder Pellets, nichtgewebtem bzw. nicht-gewebter Block, Stab, Film, Membran, Fadenfilter, Folie, Röhre oder Beschichtung; oder
- gewebter Struktur, wie ein Film, eine Membran, ein Filter, eine Hülse, ein Rohr oder eine Beschichtung besitzt.

## Revendications

1. Composition biocompatible comprenant un matériel bioactif et un matériau poreux, **caractérisée par le fait que** ledit matériel bioactif est réparti à l'intérieur dudit matériau poreux, dans laquelle
i) ledit matériel bioactif est présent sous forme de particules incluses dans ledit matériau poreux et/ou
ii) ledit matériel bioactif est présent sous forme de gel de Si issu d'une solution contenant un composé du silicium, ladite solution ayant été mise en contact avec le matériau poreux,
et **par le fait que** ledit matériau poreux est un matériau cellulosique spongieux, biorésorbable, avec des groupes hydroxyle et/ou acide carboxylique, ayant la forme d'un réservoir comprenant des particules dudit matériel bioactif ou un gel de celui-ci.

2. Composition selon la revendication 1, **caractérisée par le fait que** le matériel bioactif comprend des particules d'un verre bioactif.

3. Composition selon la revendication 1 ou 2, **caractérisée par le fait que** la solution contenant le composé du silicium est une solution de silicate alcalin.

4. Composition selon la revendication 1, **caractérisée par le fait que** le matériau poreux comprenant des particules du matériel bioactif a été immergé dans une solution contenant un composé du silicium, après quoi le silicium a été transformé en gel.

5. Composition selon la revendication 4, **caractérisée par le fait que** le matériau poreux est une éponge cellulosique, le matériel bioactif est un verre bioactif et la solution contenant le composé du silicium est une solution de silicate alcalin.

6. Composition selon l'une quelconque des revendications 1 à 5, **caractérisée par le fait que** le gel de Si est dopé avec un ou plusieurs agents capables d'affecter ses propriétés telles que la vitesse de résorption du gel de Si.

7. Composition selon l'une quelconque des revendications 1 à 6, **caractérisée par le fait que** ladite composition est renforcée avec des fibres organiques et/ou inorganiques.

8. Composition selon l'une quelconque des revendications 1 à 7, **caractérisée par le fait qu'**elle comprend un ou plusieurs agents thérapeutiquement ou biologiquement actifs.

9. Implant comprenant une composition selon l'une quelconque des revendications précédentes, **caractérisé par le fait que** ledit implant est sous forme
- de fibres,
- de granules ou pellets, de bloc, de tige, de feuille, de membrane, de filtre, de fil, de film, de tube ou de revêtement non tissés ; ou
- d'une structure tissée telle qu'une feuille, une membrane, un filtre, une chaussette, un tube ou un revêtement.

10. Procédé de préparation d'une composition selon la revendication 1, **caractérisé par le fait que** l'on
- mélange les particules de matériel bioactif avec une suspension de fibres de matériau cellulosique et une ou plusieurs substances auxiliaires nécessaires pour transformer lesdites fibres en un matériau spongieux poreux, et
- laisse lesdites fibres et la ou les substances auxiliaires former le matériau spongieux poreux.

11. Procédé selon la revendication 10, **caractérisé par le fait que** le matériau spongieux poreux est ensuite immergé dans une solution contenant un composé du silicium, après quoi le composé du silicium est transformé en gel puis le produit immergé est éventuellement acidifié, compressé et séché.

12. Utilisation d'une composition selon l'une quelconque des revendications 1 à 8 pour la fabrication d'une préparation utilisée pour le remplacement d'os et/ou le remplissage de cavités osseuses, la minéralisation de tissus tels que la dentine pour le traitement des dents sensibles, la stabilisation des dents, la stabilisation d'implants, le remplacement ou l'augmentation de cartilage ou l'utilisation en chirurgie des tissus mous.

13. Utilisation d'une composition selon l'une quelconque des revendications 1 à 8 en tant que véhicule pour l'administration de médicaments.

14. Utilisation d'une composition selon l'une quelconque des revendications 1 à 8 pour la fabrication d'une préparation destinée à des applications cliniques pour remplacer des greffes osseuses afin 1) de remplir des cavités osseuses dans tous les domaines de l'orthopédie, de la neurochirurgie, de la chirurgie en oto-rhino-laryngologie et de la dentisterie ; 2) de stimuler la guérison des fractures, en particulier dans le cas de pseudo-arthrose; 3) de créer des os spongieux dans des tissus mous à des fins de greffe ; ou 4) d'accélérer l'ostéosynthèse.

15. Dispositif destiné au traitement de dents sensibles, ledit dispositif comprenant une composition selon l'une quelconque des revendications précédentes, **caractérisé par le fait que** ledit dispositif présente la forme
- de fibres,
- de granules ou pellets, de bloc, de tige, de feuille, de membrane, de filtre, de fil, de film, de tube ou de revêtement non tissés ; ou
- d'une structure tissée telle qu'une feuille, une membrane, un filtre, une chaussette, un tube ou un revêtement.
